# EUROPÄISCHE PATENTANMELDUNG

(11) **EP 1 704 874 A1**
(43) Veröffentlichungstag der Anmeldung: **27.09.2006**
(21) Anmeldenummer: 06004519.2
(22) Anmeldetag: 06.03.2006
(51) Int. Cl.: A61L 9/04, A61L 9/12

(54) **Duftabgabesystem**

(30) Priorität: 24.03.2005 DE 102005014279
(71) Anmelder: Henkel Kommanditgesellschaft auf Aktien, 40589 Düsseldorf (DE)
(72) Erfinder: Barthel, Wolfgang, 40764 Langenfeld (DE); Mühlhausen, Hans-Georg, 40597 Düsseldorf (DE); Großmann, Barbara, 40229 Düsseldorf (DE); Canavoi, Jan, 40591 Düsseldorf (DE); Kessler, Arnd, 40789 Monheim-Baumberg (DE)

(57) **Zusammenfassung**

Duftabgabesystem (1) zur Beduftung eines geschlossenen Raumes, in dem wenigstens ein Gerät wie beispielsweise ein Computer (12) aufstellbar ist, der im Betrieb einen Luftstrom emittiert, wobei das Duftabgabesystem (1) einen Behälter (2), der eine mit Öffnungen (20) versehene Rückwand (6) und eine mit Öffnungen (21) versehene Vorderwand (5) aufweist, eine Vielzahl von im Behälter befindlichen Partikel (4), die ein vorzugsweise polymeres Trägermaterial sowie wenigstens einen Duftstoff aufweisen, und des weiteren Befestigungsmittel (16) umfasst, durch die der Behälter (2) an einer mit Lüftungsschlitzen versehenen Gehäusewand (13) des Gerätes befestigbar ist. Das Duftabgabesystem zeichnet sich dadurch aus, dass die Vorderwand (5) nach außen gewölbt und die Rückwand (6) nach innen gewölbt ist, wobei in befestigter Lage des Duftabgabesystems (1) die Rückwand (6) der Gehäusewand (13) des Gerätes zugewandt ist, so dass der Luftstrom durch die Lüftungsschlitze (14), durch die Öffnungen (20) der Rückwand (6) in den Behälter (2) gelangen und dann durch die Öffnungen (21) derVorderwand (5) in den Raum treten kann.

## Beschreibung

Die Erfindung betrifft ein Duftabgabesystem zur Beduftung eines geschlossenen Raumes.

Duftabgabesysteme weisen üblicherweise einen Behälter zur Aufnahme eines Duftstoffes auf. Bei Duftabgabesystemen, die nach der sogenannten passiven Arbeitsweise funktionieren, wird der Duftstoff permanent durch Verdunstung bei Raumtemperatur freigesetzt. Sie weisen in der Regel eine relativ lange Zeit auf, bis der freigesetzte Duft im ganzen Raum wahrgenommen wird. Die Duftabgabe erfolgt dabei permanent, auch wenn der Raum nicht benutzt wird, was zu einem unnötigen hohen Duftstoffverbrauch führt.

Des weiteren sind Duftabgabesysteme mit einer Heizvorrichtung bekannt, durch die der Duftstoff zum Verdunsten auf eine Temperatur erwärmt wird, die über der Raumtemperatur liegt. Zwar kann durch eine Steuerung der Heizvorrichtung die Freisetzung des Duftstoffes geregelt werden, jedoch muss diesen Duftabgabesystemen Energie, beispielsweise durch das Stromnetz, zugeführt werden.

Aus der US 2003/0012680 ist ein Duftabgabesystem bekannt, durch das ein geschlossener Raum, in dem ein Computer oder dergleichen aufgestellt ist, beduftet werden kann. Computer weisen üblicherweise einen Ventilator zum Kühlen eines Prozessors auf. Im Betrieb emittiert dabei der Computer einen mehr oder weniger aufgewärmten Luftstrom, der durch eine mit Lüftungsschlitzen versehene Gehäusewand des Computers nach außen tritt. Das Duftabgabesystem weist Befestigungsmittel auf, durch die es an den Lüftungsschlitzen des Computers befestigt werden kann. Der Behälter des Duftabgabesystems weist eine mit Öffnungen versehene Rückwand und eine mit Öffnungen versehene Vorderwand auf. In befestigter Lage an dem Computer liegt dabei die Rückwand auf der mit Lüftungsschlitzen versehenen Gehäusewand plan auf.

Aus der WO 85/00981 ist ein Duftabgabesystem bekannt, das mit einem Duftstoff imprägnierte Kunststoffkugeln aufweist und an mit Gebläseluft betriebene Geräte befestigt werden kann. Derartige Kunststoffkugeln, die mit einem Duftstoff imprägniert sind, können im Vergleich zu Systemen, die Duftstoffträger aus Papier oder Flies benutzen, länger verwendet werden und weisen eine gleichmäßigere Abgabecharakteristik über ihre Verwendungsdauer auf.

Der Erfindung liegt die Aufgabe zugrunde, ein weiteres Duftabgabesystem mit guten Duftabgabeeigenschaften bereitzustellen, das an einer mit Lüftungsschlitzen versehenen Gehäusewand eines Gerätes wie beispielsweise ein Computer befestigbar ist.

Die der Erfindung zugrundeliegende Aufgabe wird durch ein Duftabgabesystem mit den Merkmalen des Anspruchs 1 gelöst. Bevorzugte Ausführungsbeispiele der Erfindung können den Unteransprüchen entnommen werden.

Dadurch, dass die Rückwand nach innen gewölbt ist, entsteht in befestigter Gebrauchslage des Duftabgabesystems zwischen ihm und der mit Lüftungsschlitzen versehenen Gehäusewand des Gerätes, die üblicherweise eben ausgeführt ist, ein in etwa kalottenförmiger Raum, in den der Luftstrom durch die Lüftungsschlitze der Gehäusewand tritt. Von diesem Raum aus gelangt der Luftstrom durch die Öffnungen der nach innen gewölbten Rückwand in den Behälter, wobei, bedingt durch die Wölbung der Rückwand und durch die einzelnen Öffnungen, eine Vielzahl von Teilströmen entstehen, die nicht mehr parallel, sondern strahlenförmig auseinanderlaufen. Von dem Behälter aus gelangen die Teilströme durch die Öffnungen der nach außen gewölbten Vorderwand in den zu beduftenden Raum. Die Teilströme werden im Behälter, an den Partikeln vorbei, in Kanälen geführt die durch aneinander gereihte Zwischenräumen gebildet werden, die zwischen den Partikeln im Behälter zwangsläufig gegeben sind. Die Partikel können dabei vorzugsweise kugelförmig sein, aber auch andere Formen annehmen (zum Beispiel zylindrisch, linsenförmig, mehreckig).

Durch die nach außen gewölbte Vorderwand treten die Teilströme wiederum strahlenförmig in verschiedenen Richtungen aus dem Behälter, wobei näherungsweise davon ausgegangen werden kann, dass der Luftstrom in Richtung der Flächennormalen des entsprechenden Flächenstückes der Rückwand heraustritt, in dem sich die betreffende Öffnung befindet. Durch diese verschiedenen Strömungsrichtungen wird eine gleichmäßige Verteilung des Duftstoffes in dem zu beduftenden Raum begünstigt.

Wie oben bereits erwähnt, kann das Gerät ein Computer sein, der aufgrund der notwendigen Kühlung des Computerprozessors und gegebenenfalls anderer Computerbauteile einen erwärmten Luftstrom emittiert. Unter Gerät soll allgemein jede Vorrichtung verstanden werden, die bei ihrem Betrieb oder gewöhnlichen Einsatz einen Luftstrom verursacht. Der Luftstrom kann dabei durch einen Lüfter, einen Ventilator, eine Warmluftabfuhr oder auch durch einen Dichteunterschied verschiedene Luftströme erzeugt werden. Einige Beispiele für derartige Geräte im Bereich Haushalt, Büro und Arbeitsraum sind Klimaanlage, Laserdrucker und Heizkörper.

Die Partikel weisen ein Trägermaterial, vorzugsweise aus einem Polymer, sowie wenigstens einen Duftstoff auf. Die Partikel haben bevorzugt einen Schmelz ― oder Erweichungspunkt zwischen 30 und 150°C, insbesondere zwischen 75 und 80°C. Das polymere Trägermaterial kann beispielsweise eine Substanz aus der Gruppe der Ethylen/Vinylacetat-Copolymere, der Polyethylene niederer oder hoher Dichte (LDPE, HDPE) oder Gemische derselben, Polypropylen, ein Polyethylen/Polypropylen-Copolymer, Polyether/Polyamid-Blockcopolymer,Styrol/Butadien-(Block-)Copolymer, Styrol/Isopren-(Block-)Copolymer,Styrol/Ethylen/Butylen-Copolymer, Acrylnitril/Butadien/Styrol-Copolymer, Acrylnitril/Butadien-Copolymer, Polyetherester, Polyisobuten, Polyisopren, Ethylen/Ethylacrylat-Copolymer, Polyamid, Polycarbonat, Polyester, Polyacrylnitril, Polymethyl-methacrylat, Polyurethan oder einen Polyvinylalkohole umfassen.

In einer bevorzugten Ausführungsform enthalten die Partikel zur Beduftung von Räumen mindestens 10 Gew.-%, vorzugsweise mindestens 30 Gew.-%, und besonders bevorzugt mindestens 70 Gew.-% Ethylen/Vinylacetat-Copolymer, und besonders bevorzugt sind sie vollständig aus Ethylen/Vinylacetat-Copolymer hergestellt.

Weiter ist es möglich, dass als polymeres Trägermaterial Ethylen/Vinylacetat-Copolymer eingesetzt wird und dieses Copolymer 5 bis 50 Gew.-% Vinylacetat, vorzugsweise 10 bis 40 Gew.-% Vinylacetat und insbesondere 20 bis 30 Gew.-% Vinylacetat, jeweils bezogen auf das Gesamtgewicht des Copolymers, enthält.

Der Gewichtsanteil des/der Duftstoffe(s) in den Partikeln beträgt beispielsweise 1 bis 70 Gew.-%, vorzugsweise 10 bis 60 Gew.-%, und besonders bevorzugt 20 bis 40 Gew.-%, jeweils bezogen auf das Gesamtgewicht der Partikel.

In einer bevorzugten Ausführungsform weisen die Partikel einen mittleren Durchmesser von 0,5 bis 20 mm, vorzugsweise von 1 bis 10 mm und insbesondere von 3 bis 6 mm auf.

In einem bevorzugten Ausführungsbeispiel ist der Behälter im wesentlichen rotationssymmetrisch ausgebildet. In der Draufsicht weist dabei der Behälter einen kreisrunden Querschnitt auf. Die Rotationssymmetrie trägt dabei zu einem gleichmäßigen Austritt durch die Öffnungen in der Vorderwand bei. Die Öffnungen sind vorzugsweise in Umfangsrichtung bezogen auf die Symmetrieachse in gleichen Abständen zueinander angeordnet.

In einem bevorzugten Ausführungsbeispiel ist zwischen der Vorderwand und der Rückwand ein wulstförmiger Übergangsbereich vorgesehen, der eine ebene Auflagefläche ausbildet. Diese ebene Auflagefläche würde bei einem mit Lüftungsschlitzen versehenen Gehäuseteil eines Computers, das üblicherweise eben ausgebildet ist, plan und spaltfrei aufliegen. Dadurch wird sichergestellt, dass der Luftstrom, der durch das Duftabgabesystem abgedeckten Teil der Lüftungsschlitze nach außen tritt, vollständig durch den Behälter an den Partikeln vorbeigeführt wird.

Vorzugsweise ist der Abstand zwischen Vorderwand und Rückwand des Behälters in etwa konstant. Der in etwa konstante Abstand zwischen den beiden Wänden führt zu einer etwa konstanten Schichtdicke, der im Behälter befindlichen Partikel. Eine konstante Schichtdicke wiederum führt zu gleichlangen Strömungskanälen zwischen den Öffnungen der Rückwand und den Öffnungen der Vorderwand, so dass die Strömungsverhältnisse im gesamten Behälter näherungsweise gleich sind und jeder Partikel in erster Näherung mit einem gleichgroßen Luftstrom beaufschlagt wird, was für eine gleichmäßige Freisetzung des Duftstoffes pro Partikel sorgt.

Der Behälter kann grundsätzlich auf unterschiedliche Weise hergestellt werden, er kann beispielsweise einteilig durch einen Blasformvorgang hergestellt und dann durch eine Öffnung im Behälter mit den Partikeln befüllt werden, wobei die Öffnung nach der Befüllung anschließend verschlossen wird. Bevorzugt ist jedoch vorgesehen, dass der Behälter zweiteilig ausgebildet ist, wobei ein erster Teil die Vorderwand und ein zweiter Teil die Rückwand umfasst. Die beiden Teile des Behälters können dann auf einfache Weise im Spritzgießverfahren hergestellt werden.

Die beiden Teilen können auf unterschiedliche Weise miteinander verbunden werden. Bevorzugt sind sie mittels einer Rastverbindung miteinander verbunden.

In einem bevorzugten Ausführungsbeispiel umfassen die Befestigungsmittel wenigstens einen Magneten. Der wenigstens eine Magnet ist vorzugsweise in dem Bereich des Duftabgabesystems angeordnet, der in Gebrauchslage mit der mit Lüftungsschlitzen versehenen Gehäusewand des Gerätes zur Anlage kommt. Durch den Magneten lässt sich das Duftabgabesystem in einfacher Weise an einer metallene Gehäusewand fixieren, wobei das Duftabgabesystem durch die magnetische Kraft an dem Gehäuse gehalten wird.

Alternativ oder zusätzlich können die Befestigungsmittel einen Haken, einen Clip oder dergleichen umfassen. Damit sollen all diejenigen Befestigungsmittel eingeschlossen werden, die zu einer formschlüssigen Verbindung zwischen Duftabgabesystem und Gehäusewand des Gerätes führen.

Bevorzugt umfassen die Befestigungsmittel einen Klettverschluss und/oder einen Kleber, dem sich das Duftabgabesystem stoffschlüssig an die Gehäusewand des Gerätes anbringen lässt.

Anhand eines in den Figuren dargestellten Ausführungsbeispiels wird die Erfindung näher erläutert. Es zeigen:
- Fig. 1: ein erfindungsgemäßes Duftabgabesystem im Querschnitt;
- Fig. 2: das Duftabgabesystem der Figur 1, jedoch ohne Partikel und in einem nicht geschlossenen Zustand; und
- Fig. 3: das Duftabgabesystem, wie es an einem schematisch dargestellten Computer befestigt ist.

Ein allgemein mit 1 bezeichnetes Duftabgabesystem weist einen im wesentlichen rotationssymmetrischen Behälter 2 mit einem Aufnahmeraum 3 auf. In dem Aufnahmeraum 3 befinden sich eine Vielzahl von kugelförmigen Partikeln 4 zur Beduftung eines geschlossenen Raumes. Die Partikel 4 weisen ein polymeres Trägermaterial und einen Duftstoff in Form eines Parfumöls auf. Als Trägermaterial wird zum Beispiel das Polymergranulat aus Ethylen-Vinyl-Acetat mit dem kommerziellen Namen Elvax 150 von der Firma DuPont verwendet. Eine Parfumölbeladung von 30 bis 35 % ist mit Elvax 150 möglich.

Das polymere Trägermaterial ermöglicht eine Beladung der einzelnen Partikel 4 mit einem Parfümöl bei niedriger Temperatur (20 bis 30°C), so dass das Parfümöl durch Erhitzung nicht nachteilig verändert wird. Das Beladen der Partikel mit Parfümöl erfolgt in einem Mischer und dauert wenige Stunden. Je größer die Partikel, desto größer ist die Dauer der Beladung mit dem Parfümöl.

Der Aufnahmeraum 3 des Behälters weist eine mondsichelartige Querschnittsform mit einer nach außen gewölbten Vorderwand 5 und einer nach hinten gewölbten Rückwand 6 auf, wobei die beiden Endbereiche 7 der mondsichelartigen Querschnittsform des Aufnahmeraumes 3 abgerundet ausgebildet sind. In dem in den Figuren dargestellten Ausführungsbeispiel ist der Behälter 2 zweiteilig ausgebildet, wobei ein erster Teil 2a die nach außen gewölbte Vorderwand 5 und ein zweiter Teil 2b die nach innen gewölbte Rückwand 6 umfasst.

Der die Rückwand 6 umfassende Teil 2b des Behälters 2 weist ferner einen wulstartigen Übergangsbereich 8 auf, welcher mit einem stegartigen Randbereich 9 des ersten Teiles 2a verbindbar ist, wobei zur Verbindung bevorzugt eine Rastverbindung vorgesehen ist. Am stegartigen Randbereich 9 ist eine Rastwulst 10 vorgesehen, die mit einer an dem Randbereich 9 angeformten Rastnase 11 zusammenwirkt.

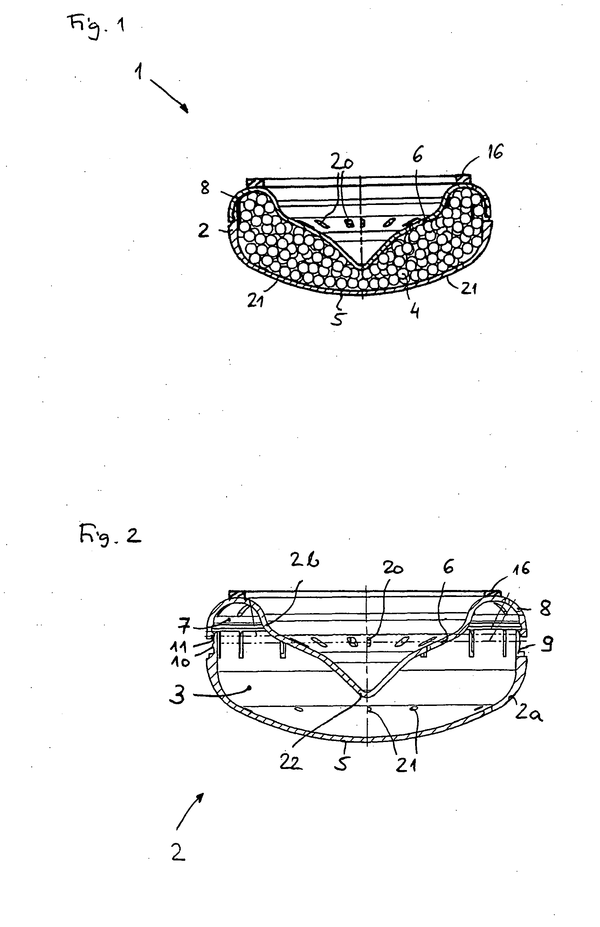

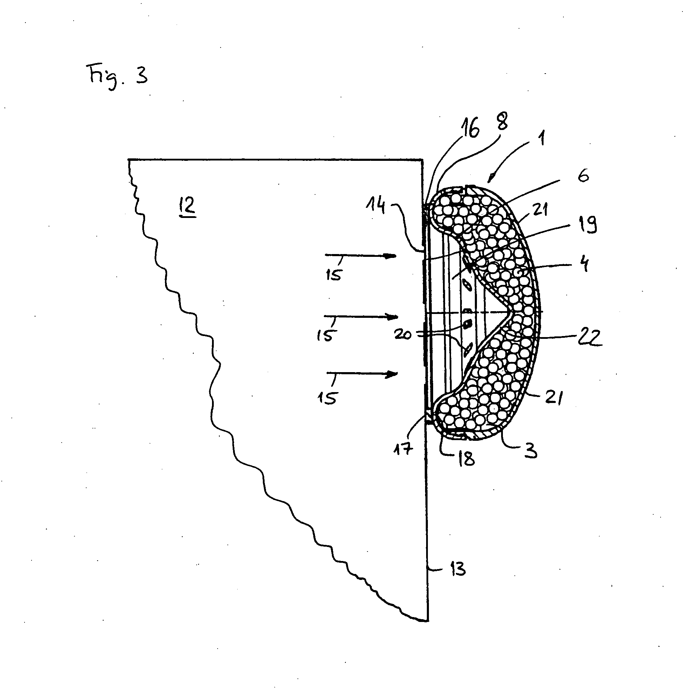

Figur 3 zeigt das Duftabgabesystem 1 wie es an einem hier nur schematisch und teilweise dargestellten Computer 12 befestigt ist. Der Computer 12 weist eine Gehäusewand 13 auf, die mit mehreren Lüftungsschlitzen 14 versehen ist. In dem Computer 12 ist ein hier nicht dargestellter Ventilator angeordnet, der zum Kühlen der im Computer 12 befindlichen Bauteile (nicht dargestellt) vorgesehen ist. Durch den Ventilator wird ein Luftstrom erzeugt, der leicht aufgewärmt in Richtung der Pfeile 15 durch die Lüftungsschlitze 14 durch die Gehäusewand 13 tritt. Das Duftabgabesystem 1 ist mittels eines ringförmigen Klebestreifens 16 an der Gehäusewand 13 befestigt. Der Klebestreifen 16 weist zwei mit einem Kleber versehene Stirnflächen 17, 18 auf, von denen die Stirnfläche 17 an der Gehäusewand 13 anliegt und die Stirnfläche 18 an dem Übergangsbereich 8 des Behälters 2 anliegt. Der Übergangsbereich 8 bildet dabei für den Klebestreifen 16 eine ebene Auflagefläche aus, so dass der Behälter mit dem Übergangsbereich 8 über seinen gesamten Umfang plan und ohne Spalt auf der ebenen Gehäusewand 13 aufliegen kann.

Nach Durchtritt durch die Lüftungsschlitze 14 gelangt der Luftstrom in einen in etwa kalottenförmigen Raum 19, der einerseits durch die Gehäusewand 13 und andererseits durch die Rückwand 6 begrenzt wird.

Von diesem kalottenförmigen Raum 19 gelangt nun der Luftstrom durch Öffnungen 20 in der Rückwand 6 in den Aufnahmeraum 3 des Behälters 2 und umströmt die in dem Aufnahmeraum befindlichen Partikel 4. Dabei nimmt der Luftstrom den in den Partikeln 4 befindlichen Duftstoff auf und transportiert diesen durch Öffnungen 21 in der Vorderwand 5 in den Raum, in dem sich der Computer 12 befindet. Durch die nach außen gewölbte Vorderwand 5 mit den dort vorgesehenen Öffnungen 21 wird der den Duftstoff tragende Luftstrom strahlenförmig in verschiedene Richtungen gelenkt, wodurch eine gute Verteilung des Duftstoffes in dem Raum begünstigt wird.

Durch die nach innen gewölbte Rückwand 6 mit ihren entsprechenden Öffnungen 20 wird der im wesentlichen parallele Luftstrom (siehe Pfeile 15) in Teilströme aufgeteilt, die, bezogen auf die Symmetrieachse des Behälters 2, mit einer radialen Komponente versehen in den Aufnahmeraum 3 strömen. Diese Teilströme werden durch eine Vielzahl von einzelnen Strömungskanälen geleitet, die sich durch die Aneinanderreihung von Zwischenräumen zwischen den einzelnen Partikeln 4 ergeben. Ein Abstand von Vorderwand 5 zur Rückwand 6 in Richtung der Durchströmung des Aufnahmeraums 3 ist dabei über dem gesamten Behälter 2 in etwa konstant, so dass in erster Näherung die Teilströme jeweils eine in etwa gleichgroße Anzahl von Partikeln umströmen. Dies führt im Behälter 2 zu einem gleichmäßigen Verbrauch des Duftstoffes.

Die nach innen gewölbte Rückwand 6 weist einen kegelförmigen Mittelbereich 22 auf. Dieser kegelförmige Mittelbereich 15 erleichtert das Auffüllen des Behälters 2 mit den Partikeln 4. Zum Befüllen des Behälters 2 wird zunächst der erste Teil 2a auf eine waagerechte Ebene gelegt und mit den Partikeln 4 befüllt. Danach wird der zweite Teil 2b des Behälters 2 auf den ersten Teil 2a gesetzt, wobei der kegelförmige Mittelbereich 22 die im inneren Bereich des Teiles 2a befindlichen Partikel verdrängt. Durch diese Verdrängung werden Partikel in die Endbereiche 7 des Teiles 2b geschoben, so dass nach Einrasten der Rastverbindung zwischen den Teilen 2a, 2b der Aufnahmeraum 3 vollständig mit Partikeln 4 befüllt ist.

Durch die Erfindung wird ein Duftabgabesystem bereitgestellt, dass eine gute Verteilung des Duftstoffes im zu beduftenden Raum begünstigt. Durch entsprechende Auswahl des Polymermaterials und des Duftstoffes kann das Duftabgabesystem so eingestellt werden, dass ein Duft in nennenswertem Umfang nur dann abgegeben wird, wenn der Computer 12 einen Luftstrom durch die Gehäusewand 13 emittiert. Somit ist es möglich, die Duftabgabe nur auf die Zeiten zu beschränken, in denen der Computer 12 eingeschaltet ist. Diese Einschaltzeiten des Computers 12 stimmen in der Regel mit den Zeiten überein, in denen sich ein Benutzer des Computers in dem zu beduftenden Raum befindet, soweit der Computer Teil eines Computer-Arbeitsplatzes ist. Dies führt zu einer effektiven Ausnutzung des Duftstoffes, da der Duftstoff nur dann abgegeben wird, wenn sich der Benutzer des Computers in dem Raum befindet.

### Bezugszeichenliste

- 1.: Duftabgabesystem
- 2.: Behälter
- 2a.: erster Teil des Behälters
- 2b.: zweiter Teil des Behälters
- 3.: Aufnahmeraum
- 4.: Partikel
- 5.: Vorderwand
- 6.: Rückwand
- 7.: Endbereich
- 8.: Übergangsbereich
- 9.: Randbereich
- 10.: Rastwulst
- 11.: Rastnase
- 12.: Computer
- 13.: Gehäusewand
- 14.: Lüftungsschlitz
- 15.: Pfeil
- 16.: Klebestreifen
- 17.: Stirnfläche
- 18.: Stirnfläche
- 19.: Raum
- 20.: Öffnung
- 21.: Öffnung
- 22.: Mittelbereich

## Patentansprüche

1. Duftabgabesystem (1) zur Beduftung eines geschlossenen Raumes, in dem wenigstens ein Gerät wie beispielsweise ein Computer (12) aufstellbar ist, der im Betrieb einen Luftstrom emittiert, wobei das Duftabgabesystem (1) einen Behälter (2), der eine mit Öffnungen (20) versehene Rückwand (6) und eine mit Öffnungen (21) versehene Vorderwand (5) aufweist, eine Vielzahl von im Behälter (2) befindlichen Partikel (4), die ein vorzugsweise polymeres Trägermaterial sowie wenigstens einen Duftstoff aufweisen, und des weiteren Befestigungsmittel (16) umfasst, durch die der Behälter (2) an einer mit Lüftungsschlitzen (14) versehenen Gehäusewand (13) des Gerätes (12) befestigbar ist, **dadurch gekennzeichnet, dass** die Vorderwand (5) nach außen gewölbt und die Rückwand (6) nach innen gewölbt ist, wobei in befestigter Lage des Duftabgabesystems (1) die Rückwand (6) der Gehäusewand (13) des Gerätes (12) zugewandt ist, so dass der Luftstrom durch die Lüftungsschlitze (14), durch die Öffnungen (20) der Rückwand (6) in den Behälter (2) gelangen und dann durch die Öffnungen (21) der Vorderwand (5) in den Raum treten kann.

2. Duftabgabesystem (1) nach Anspruch 1,
**dadurch gekennzeichnet,**
**dass** der Behälter (2) im wesentlichen rotationssymmetrisch ist.

3. Duftabgabesystem (1) nach Anspruch 1 oder 2,
**dadurch gekennzeichnet,**
**dass** zwischen Vorderwand (5) und Rückwand (6) ein wulstförmiger Übergangsbereich (8) vorgesehen ist, der eine ebene Auflagefläche ausbildet.

4. Duftabgabesystem (1) nach einem der Ansprüche 1 bis 3,
**dadurch gekennzeichnet,**
**dass** der Abstand zwischen Vorderwand (5) und Rückwand (6) in etwa konstant ist.

5. Duftabgabesystem (1) nach Anspruch 1 oder 2,
**dadurch gekennzeichnet,**
**dass** der Behälter (2) zweiteilig ausgebildet ist, wobei ein erster Teil (2a) die Vorderwand (5) und ein zweiter Teil (2b) die Rückwand (6) umfasst.

6. Duftabgabesystem (1) nach Anspruch 5,
**dadurch gekennzeichnet,**
**dass** der erste Teil (2a) und der zweite Teil (2b) durch eine Rastverbindung miteinander verbunden sind.

7. Duftabgabesystem (1) nach einem der Ansprüche 1 bis 6,
**dadurch gekennzeichnet,**
**dass** die Befestigungsmittel wenigstens einen Magneten umfassen.

8. Duftabgabesystem (1) nach einem der Ansprüche 1 bis 7,
**dadurch gekennzeichnet,**
**dass** die Befestigungsmittel einen Haken, einen Clip und/oder dergleichen umfassen.

9. Duftabgabesystem (1) nach einem der Ansprüche 1 bis 8,
**dadurch gekennzeichnet,**
**dass** die Befestigungsmittel einen Klettverschluss und/oder Kleber umfassen.
